# EUROPEAN PATENT APPLICATION

(11) **EP 0 843 019 A2**
(43) Date of publication of application: **20.05.1998**
(21) Application number: 97119495.6
(22) Date of filing: 07.11.1997
(51) Int. Cl.: C12Q 1/68

(54) **Method for determination of specific nucleic acid sequence, and a reagent therefor**

(30) Priority: 08.11.1996 JP 296963/96
(71) Applicant: KYOWA MEDEX CO., LTD., Tokyo 104 (JP)
(72) Inventor: Kawaguchi, Haruma, Yokohama-shi, Kanagawa 241 (JP); Fujimoto, Keiji, Kawasaki-shi, Kanagawa 211 (JP); Iwato, Satoko, Tokyo 150 (JP); Handa, Hiroshi, Tokyo 156 (JP); Kubota, Aiko, Yokohama-shi, Kanagawa 240 (JP); Fukui, Masanori, Himeji-shi, Hyogo 671-22 (JP)
(74) Representative: VOSSIUS & PARTNER

(57) **Abstract**

Disclosed are a method for detecting or quantitatively determining a single-stranded DNA fragment having a specific nucleic acid sequence through hybridization, and a reagent kit for the detection or determination. A carrier-bonded DNA probe comprising (a) a single-stranded DNA probe having a nucleic acid sequence complementary to the specific nucleic acid sequence of the single-stranded DNA fragment to be detected or quantitatively determined in a sample, and (b) a carrier comprising a substance with a very low adsorbance for DNA, as bonded together via or without a spacer therebetween, is hybridized with DNA fragments in the sample, and the hybridized DNA fragment is detected or quantitatively determined. The reagent kit for the detection or determination comprises (I) a reagent comprising (A) a carrier-bonded DNA probe that comprises (a) a single-stranded DNA probe having a nucleic acid sequence complementary to the specific nucleic acid sequence of a single-stranded DNA fragment to be detected or quantitatively determined in a sample, and (b) a carrier comprising a substance with a very low adsorbance for DNA as bonded together via or without a spacer therebetween, and (B) a labeled DNA probe that comprises (c) a single-stranded DNA probe having a nucleic acid sequence complementary to a partial nucleic acid sequence except the specific nucleic acid sequence of the single-stranded DNA fragment to be detected or quantitatively determined in the sample, and (d) a labeling compound as bonded together, and (II) a reagent for detecting or quantitatively determining the labeling compound.

## Description

The present invention relates to a method for simple detection or quantitative determination of specific nucleic acid sequence for early diagnosis of genotypes and gene mutations, and also to reagents therefor.

Early detection of cancer is being realized through various image diagnoses and biochemical diagnoses. In most cases, however, cancer could be discovered only after having progressed in some degree. The biopsy of pancreatic cancer is not easy, unlike that of stomach cancer and colon cancer, and it is hard to differentiate pancreatic cancer from chronic pancreatitis in many cases, often making it difficult to treat pancreatic cancer.

With the recent development of cancer-related molecular-biological studies, it has been clarified that the point mutation of K-ras codon 12 occurs at a high degree in pancreatic duct cancer which appears in most cases of human pancreatic cancer, and the detection of that point mutation through PCR (polymerase chain reaction) has become regarded as important for differential diagnosis of pancreatic cancer.

However, though having high sensitivity, PCR is expensive and troublesome, while often causing gene contamination. Therefore, PCR has not as yet been widely used for differential diagnosis of pancreatic cancer.

As a method for the purification of substances based on their specific affinity, for example, known is a method of using a carrier-bonded DNA probe to purify a protein that is specifically adsorbed on the probe DNA (JP,A, 3-61493). Also known is a method of using a DNA probe bonded to a non-specific carrier, to thereby concentrate or purify DNA or RNA that specifically bonds to the probe (J. Colloid and Interface Sci., 177, 245 (1996)).

However, no method is known for detecting or quantitatively determining specific gene fragments capable of eliminating one base mismatch through hybridization.

An object of the invention is to provide a method for detecting or quantitatively determining a single-stranded DNA fragment having a specific nucleic acid sequence through hybridization, and to provide a reagent therefor. The method and the reagent are effective in detecting or quantitatively determining point mutations related to various disorders such as cancer.

According to the invention, there is provided a method for detecting or quantitatively determining a single-stranded DNA fragment having a specific nucleic acid sequence in a sample, which comprises stringently hybridizing a carrier-bonded DNA probe that comprises (a) a single-stranded DNA probe having a nucleic acid sequence complementary to the specific nucleic acid sequence of the single-stranded DNA fragment to be detected or quantitatively determined in the sample, and (b) a carrier comprising a substance with a very low adsorbance for DNA, as bonded together via or without a spacer therebetween, with DNA fragments in the sample, followed by detecting or quantitatively determining the DNA fragment as hybridized with the carrier-bonded DNA probe.

There is also provided a method for detecting or quantitatively determining a single-stranded DNA fragment having a specific nucleic acid sequence in a sample, which comprises stringently hybridizing both (A) a carrier-bonded DNA probe that comprises (a) a single-stranded DNA probe having a nucleic acid sequence complementary to the specific nucleic acid sequence of a single-stranded DNA fragment to be detected or quantitatively determined in the sample, and (b) a carrier comprising a substance with a very low adsorbance for DNA, as bonded together via or without a spacer therebetween, and (B) a single-stranded DNA probe having a nucleic acid sequence complementary to a partial nucleic acid sequence except the specific nucleic acid sequence of the single-stranded DNA fragment to be detected or quantitatively determined in the sample, with DNA fragments in the sample, followed by detecting or quantitatively determining the DNA fragments as hybridized with the both probes (A) and (B).

There is further provided a method for detecting or quantitatively determining a single-stranded DNA fragment having a specific nucleic acid sequence in a sample, which comprises hybridizing a carrier-bonded DNA probe that comprises (a) a single-stranded DNA probe having a nucleic acid sequence complementary to the specific nucleic acid sequence of a single-stranded DNA fragment to be detected or quantitatively determined in the sample, and (b) a carrier comprising a substance with a very low adsorbance for DNA, as bonded together via or without a spacer therebetween, with DNA fragments in the sample, then treating it with an enzyme capable of cleaving the single-stranded DNA fragment, and thereafter detecting or quantitatively determining the DNA fragment as hybridized with the carrier-bonded DNA probe.

There is still further provided a carrier-bonded DNA probe, which comprises a 10-meric to 30-meric single-stranded DNA probe having a nucleic acid sequence complementary to the specific nucleic acid sequence of a single-stranded DNA fragment to be detected or quantitatively determined in a sample, and a carrier comprising a substance with a very low adsorbance for DNA, as bonded together via or without a spacer therebetween.

There is still further provided a reagent kit for detecting or quantitatively determining a single-stranded DNA fragment having a specific nucleic acid sequence in a sample, which comprises (I) a reagent comprising (A) a carrier-bonded DNA probe that comprises (a) a single-stranded DNA probe having a nucleic acid sequence complementary to the specific nucleic acid sequence of a single-stranded DNA fragment to be detected or quantitatively determined in the sample, and (b) a carrier comprising a substance with a very low adsorbance for DNA as bonded together via or without a spacer therebetween, and (B) a labeled DNA probe that comprises (c) a single-stranded DNA probe having a nucleic acid sequence complementary to a partial nucleic acid sequence except the specific nucleic acid sequence of the single-stranded DNA fragment to be detected or quantitatively determined in the sample, and (d) a labeling compound as bonded together, and (II) a reagent for detecting or quantitatively determining the labeling compound.

There is still further provided a labeled, carrier-bonded DNA probe, which comprises (a) a labeled single stranded DNA probe comprising a nucleic acid sequence complementary to the specific nucleic acid sequence of a single-stranded DNA fragment to be detected or quantitatively determined in a sample, and a labeling compound as bonded together, and (b) a carrier comprising a substance with a very low adsorbance for DNA, as bonded together via or without a spacer therebetween.

There is still further provided a reagent kit for detecting or quantitatively determining a single-stranded DNA fragment having a specific nucleic acid sequence in a sample, which comprises (I) a reagent comprising a labeled, carrier-bonded DNA probe that comprises (a) a labeled, single-stranded DNA probe comprising a nucleic acid sequence complementary to the specific nucleic acid sequence of a single-stranded DNA fragment to be detected or quantitatively determined in the sample, and a labeling compound, as bonded together and (b) a carrier comprising a substance with a very low adsorbance for DNA as bonded together via or without a spacer therebetween, (II) a reagent comprising an enzyme capable of cleaving the single-stranded DNA, and (III) a reagent for detecting or quantitatively determining the labeling compound.

Fig. 1 shows a calibration curve for the amount of DNA.

Fig. 2 shows DNA dissolution curves for a 20-mer probe, in which -●- indicates the combination of sequence No. 2 and Sequence No. 1 (completely complementary sequences), and -▲- indicates the combination of Sequence No. 2 and Sequence No. 4 (one base mismatched).

Fig. 3 shows DNA dissolution curves for a 70-mer probe, in which the -●- indicates the combination of Sequence No. 9 and Sequence No. 6 (completely complementary sequences), and -O- indicates the combination of Sequence No. 9 and Sequence No. 7 (one base mismatched).

Fig. 4 shows the difference in relative absorbance as a function of probe length.

In the invention, used is a carrier-bonded DNA probe that comprises (a) a single-stranded DNA probe having a nucleic acid sequence complementary to the specific nucleic acid sequence of a single-stranded DNA fragment to be detected or quantitatively determined in a sample, and (b) a carrier comprising a substance with a very low adsorbance for DNA, as bonded together via or without a spacer therebetween.

The shape of the carrier is not specifically defined. For example, the carrier includes plate-shaped carriers and carrier grains. Preferred are carrier grains, to which a larger amount of the DNA probe can be bonded per unit volume of the carrier. The grain diameter is preferably 0.01 to 100 µm, more preferably 0.05 to 20 µm. Desirably, the surface of the carrier is coated with a substance with a very low adsorbance for DNA. The substance with a very low adsorbance for DNA is a substance having many hydrophilic functional groups on its surface, which may be any of natural polymers or synthetic polymer compounds.

The natural polymers may be, for example, polysaccharides such as chitosan and hyaluronic acid. The synthetic polymer compounds may be, for example, those having hydroxyl groups and/or amido groups in the side chains.

As monomers constituting those synthetic polymer compounds, mentioned are ethylene oxide-containing methacrylates such as ethylene glycol methacrylate and triethylene glycol methacrylate; hydroxyl-containing methacrylates such as hydroxymethyl methacrylate and hydroxypropyl methacrylate; epoxy-containing methacrylates such as glycidyl methacrylate; alkyl methacrylates such as methyl methacrylate and ethyl methacrylate; monoethylenic unsaturated amides such as acrylamide, methacrylamide, diacetacrylamide and N-hydroxymethylacrylamide; and ethylenic unsaturated nitriles such as acrylonitrile and methacrylonitrile.

The synthetic polymer compounds may be homopolymers of one of those monomers or copolymers of two or more of those monomers. The synthetic polymer compounds may be hydrophilic synthetic polymer compounds. Examples of preferred polymer compounds are polymers of glycidyl methacrylate. The synthetic polymer compound may form at least the surface layer of the carrier, while the inside of the carrier may be a hydrophobic polymer such as a polystyrene or polyvinyl compound. Preferably, the surface of the carrier is designed to have many epoxy groups. An example of such carrier is a carrier grain having a core/shell structure of polystyrene/polyglycidyl methacrylate.

The polymer compounds can be produced in any known methods depending on the type of the monomers used and the type of the polymer compounds to be produced.

Where polymer grains are desired to be produced, employable is suspension polymerization or emulsion polymerization, etc. For the suspension polymerization, a dispersion stabilizer may be added to the reaction system. The dispersion stabilizer includes water-soluble polymers, such as polyacrylamide and its limited hydrolysates, polyacrylic acid, hydroxypropyl cellulose, ethyl cellulose, methyl cellulose, polyvinyl alcohol, and polyvinyl acetate. The polymerization initiator for the suspension polymerization is not specifically defined, including azo-type initiators such as azobisisobutyronitrile and 2,2'-azobis(2-aminopropane) dihydrochloride; and peroxides such as benzoyl peroxide. The polymerization initiator for the emulsion polymerization is not also specifically defined, and may be any and every one employable in any ordinary emulsion polymerization. The emulsion polymerization may be effected in any of batchwise, semi-batchwise or continuous systems. The emulsion polymerization is preferably soap-free emulsion polymerization comprising water, monomer and polymerization initiator, in order that the surfaces of the polymer grains formed are kept cleaned and that any adsorbing substances are not introduced into the polymerization system. More preferred is two-stage, soap-free emulsion polymerization.

The spacer as referred to herein is to bond the carrier comprising a substance with a very low adsorbance for DNA, to the single-stranded DNA probe having a nucleic acid sequence complementary to the specific nucleic acid sequence of a single-stranded DNA fragment to be detected or quantitatively determined in a sample. The spacer includes polyethylene glycol diglycidyl ether chains, single-stranded DNA chains, and single-stranded RNA chains. The length of the polyethylene glycol diglycidyl ether chain is preferably 1 to 10, more preferably 1 to 5, in terms of the number of the ethylene units constituting it. The single-stranded DNA chain and the single-stranded RNA chain to be used as the spacer may have any base sequence, but preferably has an amino base, such as adenine (A), guanine (G) or cytosine (C), at its terminals. Regarding their length, the single-stranded DNA chain and the single-stranded RNA chain are preferably 5- to 40-mer, more preferably 10- to 20-mer.

Regarding a length of the single-stranded DNA probe having a nucleic acid sequence complementary to the specific nucleic acid sequence of a single-stranded DNA fragment to be detected or quantitatively determined in a sample, the DNA fragment is comprised of preferably 10 to 50 bases, more preferably 10 to 30 bases, even more preferably 15 to 25 bases.

The specific nucleic acid sequence of a single-stranded DNA fragment to be detected or quantitatively determined in a sample may include DNA fragments derived from various character-related genes and disease-related genes. The character-related genes include PS1 (priserinine 1) gene, PS2 (priserinine 2) gene, APP (beta-amyloid precursor protein) gene, lipoprotein gene, HLA (human leukocyte antigen) gene, hepatitis virus C gene, hepatitis virus B gene, hMSH2 gene, etc. The disease-related genes include K-ras gene, p53 gene. Variations of K-ras gene are described in, for example, Jpn. J. Cancer Res., 84, 961 (1993); ibid., 85, 147 (1994); ibid., 85, 1240 (1994); ibid., 85, 1005 (1994); ibid., 86, 1150 (1995); ibid., 86, 737 (1995); ibid., 87, 466 (1996); ibid., 87, 1056 (1996); and ibid., 87, 793 (1996). Variations of p53 gene are described in, for example, Jpn. J. Cancer Res., 85, 1087 (1994); ibid., 85, 1247 (1994); ibid., 86, 1143 (1995); ibid., 86, 57 (1995); ibid., 86, 174 (1995); ibid., 86, 730 (1995); and ibid., 87, 930 (1996). hMSH2 gene is described in, for example, Jpn. J. Cancer Res., 87, 279 (1996).

Table 1 and Table 2 show some examples of partial variations of the nucleic acid sequence of K-ras gene and p53 gene, which are known to be caused by carcinogenesis. A single-stranded DNA fragment that is complementary to a specific nucleic acid sequence comprising any of those known variant sequences may be the probe DNA nucleic acid sequence for use in the invention.

**Table 1**

| Variations of K-ras Sequence | | | | |
|---|---|---|---|---|
| Exon | Codon | Normal Sequence | | Variant Sequence |
| 1 | 12 | GGT | → | GAT |
| 1 | 12 | GGT | → | GTT |
| 1 | 12 | GGT | → | TGT |
| 1 | 12 | GGT | → | GCT |
| 1 | 12 | GGT | → | CGT |
| 1 | 12 | GGT | → | AGT |
| 1 | 13 | GGC | → | GCC |

**Table 2**

| Variations of p53 Sequence | | | | |
|---|---|---|---|---|
| Exon | Codon | Normal Sequence | | Variant Sequence |
| 5 | 157 | GCT | → | CTC |
| 5 | 157 | GTC | → | TTC |
| 5 | 157 | GTC | → | ATC |
| 6 | 198 | GAA | → | CAA |
| 5 | 176 | TGC | → | TAC |
| 5 | 175 | CGC | → | CAC |
| 8 | 266 | GGA | → | GAA |
| 6 | 193 | CAT | → | CGT |
| 7 | 249 | AGG | → | ATG |
| 6 | 220 | TAT | → | TGT |
| 7 | 248 | CGG | → | TGG |
| 8 | 273 | CGT | → | CAT |
| 7 | 244 | GGC | → | TGC |
| 7 | 236 | TAC | → | AAC |
| 6 | 229 | ACT | → | ATT |
| 7 | 246 | CGC | → | CTC |
| 7 | 157 | GCC | → | GTC |
| 7 | 242 | TGC | → | TTC |
| 7 | 249 | AGG | → | AGT |
| 8 | 282 | CGG | → | TGG |
| 6 | 199 | CCG | → | CTG |
| 9 | 324 | GAT | → | AGT |

Table 3 and Table 4 show partial nucleic acid sequences of the gene of human hepatitis virus C and human leukocyte antigen. A single stranded DNA fragment that is complementary to a specific nucleic acid sequence comprising any of those sequences may be the probe DNA for use in the invention.

**Table 3**

| Human Hepatitis Virus C | | |
|---|---|---|
| Genotype | Sequence | |
| I | GGATAGGCTG ACGTCTACCT | (Sequence Number 10) |
| II | GAGCCATCCT GCCCACCCCA | (Sequence Number 11) |
| III | CCAAGAGGGA CGGGAACCTC | (Sequence Number 12) |
| IV | ACCCTCGTTT CCGTACAGAG | (Sequence Number 13) |
| V | GCTGAGCCCA GGACCGGTCT | (Sequence Number 14) |

**Table 4**

| Human Leukocyte Antigen | | |
|---|---|---|
| Type | Sequence | |
| DRB1 DR1 | TTCTTGTGGC AGCTTAAGTT | (Sequence Number 15) |
| DRB1 DR2 | TTCCTGTGGC AGCCTAAGAG G | (Sequence Number 16) |
| DRB1 DR4 | GTTTCTTGGA GCAGGTTAAA C | (Sequence Number 17) |
| DRB1 DR7 | AGTTCCTGGA AAGACTCTTC T | (Sequence Number 18) |
| DRB1 DR10 | GGTTGCTGGA AAGACGCGTC C | (Sequence Number 19) |
| DQB1 DQ2 | GATTCCCCGC AGAGGATTTC G | (Sequence Number 20) |
| DQB1 DQ4 | CACCTGCAGT GCGGAGCTCC AACTGGTA | (Sequence Number 21) |

As described above, the sequence of the single-stranded DNA probe may be designed on the basis of those known nucleic acid sequences. Nucleic acid sequences capable of being favorably detected or quantitatively determined in the invention are, for example, known nucleic acid sequences with point mutation. In those cases, the position of the nucleic acid sequence that is complementary to the mutant nucleic acid sequence, in the DNA probe is preferably inside from the both terminals by 3 bases or more, and is more preferably within 2 bases from the center.

The single-stranded DNA probe having a nucleic acid sequence complementary to the specific nucleic acid sequence of a single-stranded DNA fragment to be detected or quantitatively determined in a sample can be produced through chemical DNA synthesis in a solid phase process using a carrier such as silica, in an automatic DNA synthesizer or the like. In that process, for example, a nucleotide derivative, in which the amino group in the base moiety and the 5'-OH in the ribose moiety are protected while diisopropylphosphoamidite is bonded to the 3'-OH in the ribose moiety, is used as the reaction substrate. The protecting group for the amino group in the base moiety includes benzoyl and isobutyl groups. The protecting groups for the 5'-OH group in the ribose moiety includes a dimethoxytrityl group. In accordance with the intended base sequence to be produced, a nucleotide of any of adenine, thymidine (T), cytosine and guanine, of which the amino group and the 5'-OH group have been protected, is fixed onto a support via its 3'-OH group, and put into a column. In the column, the nucleotide thus-fixed is treated with an acid to remove the protecting trityl group to deprotect the 5'-OH group, and thereafter a suitable nucleotide derivative having a phosphoamidite group at the 3'-terminal is added to the deprotected nucleotide in the presence of a suitable condensing agent such as tetrazole. Then, iodine and water are added to the reaction system, whereby the bonding of the two is converted into a stable triphosphate bond. This cycle of de-tritylation and condensation is repeated, and the resulting condensate product is finally deprotected and separated from the support by the treatment with ammonia. As a result of this process, obtained is the intended DNA probe. In order to obtain a DNA probe having a spacer which is a single-stranded DNA, the sequence of the spacer may be produced subsequently to the production of the DNA probe.

Described hereinunder is the production of the carrier-bonded DNA probe, which comprises (a) a single-stranded DNA probe having a nucleic acid sequence complementary to the specific nucleic acid sequence of a single-stranded DNA fragment to be detected or quantitatively determined in a sample, and (b) a carrier comprising a substance with a very low adsorbance for DNA, as bonded together via or without a spacer therebetween. Depending on the type of the spacer and the type of the carrier, the method of bonding the carrier, the spacer and the single-stranded DNA probe can be quantitatively determined. In general, the DNA probe is bonded to the carrier or to the spacer through the bonding of the amino group in the terminal base of the DNA probe to the epoxy, carboxyl, aldehyde or hydroxyl group in the carrier or in the spacer.

Where the spacer is a single-stranded DNA and the carrier has epoxy groups in its surface, the DNA probe is bonded to the carrier via the spacer as follows. In the same manner as in the DNA synthesis mentioned hereinabove, a DNA probe having a DNA spacer, and a single-stranded DNA having a nucleic acid sequence complementary to the probe but not having a spacer sequence are separately prepared. Then, the two are hybridized to give a double-stranded DNA, in which the amino groups of the bases in the nucleic acid sequence to be detected or quantitatively determined are protected, and thereafter the amino group of the base in the free spacer moiety is bonded to the epoxy group in the carrier. After having been thus bonded to the carrier, the single-stranded DNA having a nucleic acid sequence complementary to the probe but not having a spacer sequence is removed from the double-stranded DNA. As a result, obtained is the intended, carrier-bonded DNA probe.

The hybridization may be carried out in a solution optionaly containing any of formamide, salts, proteins and stabilizers and buffer. The solution for the hybridization is hereinafter referred to as hybridization solution. In the solution, the formamide concentration may be 0 to 60%, preferably 10 to 50%, more preferably 20 to 30%. The salts include inorganic salts such as sodium chloride and potassium chloride; and salts of organic acids such as sodium citrate and sodium oxalate. The salt concentration may be 0 to 2.0 M, preferably 0.15 to 1.0 M. The protein may be, for example, serum albumin. The stabilizer may be, for example, Ficol. The buffer may be, for example, a phosphate buffer, and its concentration is preferably 1 to 100 mM. The hybridization of the two complementary DNAs may be attained by adding the synthesized, single-stranded DNAs to the hybridization solution to have the same molar concentration, then heating the solution at a temperature between 60 and 90°C for 1 to 60 minutes, and then gradually cooled to a temperature between 0 and 40°C over a period of 1 to 24 hours. Thus is obtained a partially double-stranded DNA having a single-stranded polynucleotide spacer.

The bonding of the partially double-stranded DNA to a carrier may be effected as follows: Epoxy-having carrier grains are washed with 1 to 100 mM phosphate buffer to thereby equilibrate the grains. Next, the carrier grains are mixed with the partially double-stranded DNA having a single-stranded polynucleotide spacer, which has been prepared in the above, in the same buffer as that used for the washing of the grains, and kept at a temperature between 20 and 50°C for 5 to 50 hours. The non-reacted DNAs are removed through washing with an aqueous solution containing a salt such as 1 to 3 M sodium chloride, and the non-reacted epoxy groups still remaining on the carrier grains are cleaved with a Tris-HCl buffer as added thereto, while leaving the carrier grains at room temperature for 5 to 50 hours. Thus is obtained a carrier-bonded, double-stranded DNA.

This carrier-bonded, double-stranded DNA is washed in the hybridization solution at a temperature between 70 and 90°C to obtain the intended, carrier-bonded, single-stranded DNA. To wash the carrier-bonded, double-stranded DNA in the hybridization solution, the hybridization solution containing the DNA is subjected to centrifugation several times at a temperature between 70 and 90°C and under a several thousands to several tens of thousands gravity.

Where the spacer is polyethylene glycol diglycidyl and the carrier has epoxy groups in its surface, the DNA probe may be bonded to the carrier as follows. Ammonium chloride or hexamethylenediamine hydrochloride is added to the carrier grains in an amount of 10 to 100 equivalents based on the epoxy groups of the carrier, and reacted at a temperature between 60 and 70°C and at a pH between 10 and 12 for 0.5 to 2 days, to thereby aminate the surfaces of the carrier grains. After the reaction, the grains are washed through centrifugation and, if desired, dialyzed against ion-exchanged water to thereby remove the non-reacted ammonium hydroxide or hexamethylenediamine hydrochloride. Polyethylene glycol diglycidyl is added to the carrier thus having amino groups introduced thereinto, in an amount of 50 to 200 equivalents based on the amino groups of the carrier, and reacted at a pH between 10 and 12 and at a temperature between 20 and 40°C for 0.5 to 2 days, whereby polyethylene glycol diglycidyl is bonded to the carrier. The spacer is bonded to the carrier in that manner. The bonding of the epoxy groups of the spacer to the probe DNA may be attained in the manner mentioned above.

Described hereinunder is the production of the single-stranded DNA fragment having the specific nucleic acid sequence to be detected or quantitatively determined in the invention. The specimen to be applied to the intended detection or quantitative determination may be prepared from various organs, tissues and blood through known DNA extraction. The isolated DNA is cleaved with specific restriction endonucleases to give a single-stranded DNA fragment containing the specific nucleic acid sequence to be detected or quantitatively determined and having a length of 10- to 200-mer, preferably 20- to 100-mer. For example, where a DNA as extracted from cells is processed with restriction endonucleases DdeI and HinfI, prepared is a DNA fragment (Sequence No.7), which contains K-ras codon 12 and having a length of 70 bases. Hereinafter, the sequence represented by Sequence No. 7 is referred to as K-ras sequence. The present invention requires the single-stranded DNA fragment having the specific nucleic acid sequence to be detected or quantitatively determined through DNA fragmentation.

Depending on the detective or quantitative determination method employed, the DNA to be labeled with a labeling compound may be any of composite DNA comprised of all DNA fragments in a sample, a single-stranded DNA probe having a nucleic acid sequence complementary to a partial nucleic acid sequence except the specific nucleic acid sequence of a single-stranded DNA fragment to be detected or quantitatively determined in a sample, or the single-stranded DNA probe moiety of a carrier-bonded DNA probe composed of a single-stranded DNA probe having a nucleic acid sequence complementary to the specific nucleic acid sequence of a single-stranded DNA fragment to be detected or quantitatively determined in a sample, and a carrier comprising a substance with a very low adsorbance for DNA, as bonded together via or without a spacer therebetwen.

Hereinunder, "labeled DNA probe" means a single-stranded DNA probe which has a nucleic acid sequence complementary to a partial nucleic acid sequence except the specific nucleic acid sequence of a single-stranded DNA fragment to be detected or quantitatively determined in a sample and which has been labeled with a labeling compound. Hereinunder, "labeled, carrier-bonded DNA probe" means a carrier-bonded DNA probe which is composed of (a) a single-stranded DNA probe having a nucleic acid sequence complementary to the specific nucleic acid sequence of a single-stranded DNA fragment to be detected or quantitatively determined in a sample, and (b) a carrier comprising a substance with a very low adsorbance for DNA, as bonded together via or without a spacer therebetween, and which has been labeled with a labeling compound at its single-stranded probe moiety.

A DNA-labeling compound may be any one capable of being directly or indirectly detected or quantitatively determined. For example, employable are thermally-stable antigens and biotin, which may be bonded to DNAs through covalent bonding. Specifically, the labeling compound for the labeled DNA probe and the labeled, carrier-bonded DNA includes thermally-stable labeling compounds such as radioactive isotopes, color reagents, fluorescence reagents, and luminescence reagents. Especially preferred is biotin.

Now referred to is the bonding of biotin to DNAs. To biotinylate the 3'-terminal of a DNA, for example, employable is any of a 3'-terminal labeling method, a bio-bridge method and a nick translation method, in which is used a biotin-bonded deoxyribonucleoside triphosphate for the enzymatic bonding of biotin to the DNA. The biotin-bonded deoxyribonucleoside triphosphate includes Bio-11-dUTP, Bio-16-dUTP and Bio-11-dCTP, which are commercially available from Enzo Co. Where synthetic DNAs are labeled with biotin, employable is a cyanoethylphosphoamidite method using an automatic synthesizer, in which 5'-terminal biotinylated DNAs are formed . The biotinylated DNAs can be purified through gel filtration using, for example, a spin column charged with Sephadex G-50.

The reagent to be used for detecting or quantitatively determining the labeling compound may be any one capable of detecting or quantitatively determining the labeling compound. Where biotin is used as the labeling compound, the reagent system to be used for detecting or quantitatively determining the labeling compound comprises a enzyme-labeled avidin and a reagent for detecting or quantitatively determining the activity of the labeling enzyme. The labeling enzyme includes peroxidase and alkaline phosphatase.

Where the labeling enzyme is peroxidase, the reagent for detecting or quantitatively determining the activity of the labeling enzyme includes a reagent comprising hydrogen peroxide and any of color reagents such as 3,3'-diaminobenzene, o-dianisidine, 4-methoxy-1-naphthol, 2,2'-azino-bis(3-ethylbenzothiazoline)-6-sulfonic acid (ABTS), 10-N-methylcarbamoyl-3,7-dimethylamino-10H-phenothiazine (MCDP), 10-N-carboxymethylcarbamoyl-3,7-dimethylamino-10H-phenothiazine (CCAP), sodium N-(carboxymethylaminocarbonyl)-4,4'-bis(dimethylamino)diphenylamine (DA-64), 4,4'-bis(dimethylamino)diphenylamine, and bis[3-bis(4-chlorophenyl)methyl-4-dimethyl-aminophenyl]amine (BCMA); a reagent comprising hydrogen peroxide and any of fluorescence reagents such as homo-vanillic acid, p-hydroxyphenylacetic acid, and diacetylfluorescein derivatives (e.g., diacetylfluorescein, diacetyldichlorofluorescein); and a reagent comprising hydrogen peroxide and any of luminescence reagents such as luminol, isoluminol, pyrogallol, and bis(2,4,6-trichlorophenyl) oxalate.

In addition, also employable are color reagents of 1-naphthol derivatives such as 4-methoxy-1-naphthol; lanthanide fluorescence reagents such as europium; and luminescence reagents such as acridinium.

Where the labeling enzyme is alkaline phosphatase, the reagent for detecting or quantitatively determining the activity of the labeling enzyme includes a reagent comprising phosphate ester such as paranitrophenyl phosphates and luminescence reagents such as adamantyloxetane derivatives.

As the reagents for detecting or quantitatively determining alkaline phosphatase, especially preferred is a reagent comprising NADP, INT-violet, NADH, ethanol, diaphorase, and alcohol dehydrogenase.

Those reagents for detecting or quantitatively determining the activity of such enzymes may contain, if desired, buffers, other substrates, other enzymes, surfactants and enzyme stabilizers. If also desired, those enzyme activity-detecting or quantitatively determining reagents may be in the form a kit comprising two or more different reagents.

The enzyme to cleave single-stranded DNAs includes nucleases such as S1 nuclease and mango bean nuclease.

It is desired that the hybridization solution mentionedabove is contained in the reagent comprising (A) a carrier-bonded DNA probe that comprises (a) a single-stranded DNA probe having a nucleic acid sequence complementary to the specific nucleic acid sequence of a single-stranded DNA fragment to be detected or quantitatively determined in a sample, and (b) a carrier comprising a substance with a very low adsorbance for DNA as bonded together via or without a spacer therebetween, and (B) a labeled DNA probe that comprises (c) a single-stranded DNA probe having a nucleic acid sequence complementary to a partial nucleic acid sequence except the specific nucleic acid sequence of the single-stranded DNA fragment to be detected or quantitatively determined in the sample, and (d) a labeling compound as bonded together with no spacer; and also contained in the reagent comprising a labeled carrier-bonded DNA probe, which comprises (C) a labeled single-stranded DNA probe having a nucleic acid sequence complementary to the specific nucleic acid sequence of a single-stranded DNA fragment to be detected or quantitatively determined in a sample, and a labeling compound as bonded together with no spacer, and (D) a carrier comprising a substance with a very low adsorbance for DNA, as bonded together via or without a spacer therebetween.

Now described hereinunder is the method for detecting or quantitatively determining a single-stranded DNA fragment having a specific nucleic acid sequence in a sample, which comprises hybridizing the carrier-bonded DNA probe that comprises (a) a single-stranded DNA probe having a nucleic acid sequence complementary to the specific nucleic acid sequence of the single-stranded DNA fragment to be detected or quantitatively determined in the sample, and (b) a carrier comprising a substance with a very low adsorbance for DNA, as bonded together via or without a spacer therebetween, with DNA fragments in the sample, followed by detecting or quantitatively determining the DNA fragment as hybridized with the carrier-bonded DNA probe. The hybridization in the method shall be effected using the hybridization solution mentioned above under stringent conditions as described below. At first, the carrier-bonded DNA probe and the sample containing DNA fragments are added to the hybridization solution, then heated at a temperature between 70 and 90°C, preferably between 80 and 85°C, for 1 to 20 minutes, preferably for 5 to 15 minutes, and thereafter gradually cooled to a temperature between 20 and 50°C, preferably between 20 and 30°C, over a period of 0.5 to 24 hours, preferably 1 to 6 hours. Next, the reaction system is washed, for example, through centrifugation when the carrier used is a carrier grain, or through simple washing when the carrier used is a plate-shaped one, whereby the non-hybridized DNA fragments are removed. The detection or quantitative determination of the DNA fragment as hybridized with the carrier-bonded DNA probe may be effected directly while the DNA fragment has been still hybridized with the carrier-bonded probe, or after the DNA fragment is separated from the carrier-bonded probe.

The detection or quantitative determination of the hybridized DNA fragment is preferably effected using a labeling compound.

Mentioned is the method of detecting or quantitatively determining the intended DNA fragment, in which all DNAs in the sample are labeled. First, all DNA fragments in the sample are labeled in the same manner as above. Next, the carrier-bonded DNA probe is hybridized with the labeled DNAs. The hybridization must be effected under stringent conditions as in the above. To detect or quantitatively determine the hybridized DNA fragment, the non-hybridized DNA fragments are first removed and thereafter the labeling compound bonding to the DNA fragment as hybridized with the carrier-bonded DNA probe is detected or quantitatively determined.

Mentioned is the method of detecting or quantitatively determining the intended DNA fragment, in which a labeled DNA probe is used. First, the sample DNAs are hybridized with both the carrier-bonded DNA probe and the labeled DNA probe under stringent conditions as described above. Next, the sample DNA fragments hybridized with the carrier-bonded DNA probe are collected, for example, through centrifugation. To detect or quantitatively determine the hybridized DNA fragment, the labeling compound bonding to thesingle-stranded DNA probe as hybridized with the sample DNA fragment as hybridized with the carrier-bonded DNA probe is detected or quantitatively determined.

Mentioned is the method of detecting or quantitatively determining the intended DNA fragment, in which a labeled, carrier-bonded DNA probe is used. First, the sample DNAs and the labeled, carrier-bonded DNA are hybridized. The condition for the hybridization is not specifically defined, so far as the completely complementary DNA fragment is indispensably hybridized with the probe. For example, the hybridization may be effected under the condition under which DNA fragments with one base mismatch may be hybridized, so far as the indispensable hybridization of the completely complementary DNA fragment is attained. Next, an enzyme capable of cleaving single-stranded DNAs, such as S1 nuclease, is applied to the reaction system, whereby the labeled, carrier-bonded DNA probe with no double-stranded DNA is digested. Also, the labeled, carrier-bonded DNA probe, with which not completely complementary DNA fragments have been hybridized to give a partially single-stranded probe DNA, is digested. To detect or quantitatively determine the completely-hybridized DNA fragment, the labeling compound of the labeled, carrier-bonded DNA is detected or quantitatively determined. In this method of using the labeled, carrier-bonded DNA, it is desirable that the DNA probe is directly bonded to the carrier with no spacer therebetween, or, if a spacer is used to bond the probe and the carrier, the spacer is preferably not a DNA chain. For example, the spacer to be used is preferably a polyethylene glycol diglycidyl ether chain such as that mentioned above. In this method, even if the sample to be tested comprises some mismatched DNA fragments, the mismatched DNA fragments in a sample can be eliminated from the hybridization system. Therefore, this method is advantageous in that only the DNA having a nucleic acid sequence completely complementary to the probe DNA in a sample can be detected.

In the invention, a calibration curve is prepared, using a quantitatively predetermined amount of a DNA fragment to be detected or quantitatively determined. On the basis of the calibration curve thus-prepared, obtained is a concentration of a DNA fragment in a sample.

Mentioned hereinunder is the method of detecting or quantitatively determining a labeling compound. Where biotin is used as the labeling compound, for example, an enzyme-labeled avidin is bonded to biotin, and thereafter the enzymatic activity of the enzyme bonded to avidin that has been bonded to biotin is detected or quantitatively determined, whereby the labeling compound, biotin, is detected or quantitatively determined.

The bonding between biotin and the enzyme-labeled avidin may be attained, for example, by keeping the two in a solution that optionally comprises any of surfactants, salts and buffers, at room temperature for 0.5 to 2 hours. After this, the enzyme-labeled avidin that has not been bonded to biotin is removed through centrifugation or simple washing.

The activity of the enzyme can be detected or quantitatively determined in any ordinary manner, for example, using any of the above-mentioned, enzymatic activity-detecting or quantitatively determining reagents.

Where alkaline phosphatase is used as the labeling enzyme of the enzyme-labeled avidin to be bonded to biotin, preferably employed is a method of using a sensitization system that comprises alcohol dehydrogenase and diaphorase (see Ann. Biol. Clin., 47, 527 (1989)) to detect or quantitatively determine the enzymatic activity of the labeling enzyme. In this method, the alcohol dehydrogenase is reacted with ethanol and NAD formed from NADP in the presence of the alkaline phosphatase to thereby give NADH, and the diaphorase is reacted with NADH thus-formed and INT-violet to give a dye such as formazan. The dye is detected or quantitatively determined through colorimetry at 492 nm.

Now, the invention is described in more detail with reference to the following Examples, which, however, are not intended to restrict the scope of the invention.

### Example 1: Production of Carrier-bonded DNA Probe

### (1) Production of Single-stranded DNAs:

Using a DNA synthesizer, prepared were the following DNA chain having a K-ras mutant sequence, which is the same as the bases from the 20^{th} to the 39^{th} of Sequence No. 6:
5'-GTTGGAGCTC GTGGCGTAGG-3' (20-mer; Sequence No. 1 with a point mutation C in position 10); and
the following DNA having 10 G bases next to the sequence complementary to Sequence No. 1:
5'-GGGGGGGGGG CCTACGCCAC GAGCTCCAAC-3' (30-mer; Sequence No. 2).

### (2) Production of Carrier:

1.2 g of styrene, 1.0 g of glycidyl methacrylate, and 0.04 g of divinylbenzene were put into a 200 ml four-neck flask equipped with a stirrer, to which was added 110 ml of water to make them dispersed in water. This was kept in a thermostat at 70°C, and purged with nitrogen gas. With stirring the dispersion in the flask at 200 rpm, 0.06 g of 2,2'-azobis(2-aminopropane) dihydrochloride, a polymerization initiator, was added thereto, and the monomers were polymerized. After 2 hours, 0.3 g of glycidyl methacrylate was added thereto and further reacted for 22 hours. The reaction mixture was centrifuged (30,000 g, 10 minutes), and the resulting precipitate was purified by washing it three times with distilled water to obtain carrier grains. The grains prepared herein are referred to as SG grains. Those were in mono-dispersion, and had a grain size of about 0.2 µm.

### (3) Fixation of DNA onto Carrier:

The SG grains prepared in (2) were equilibrated through centrifugation with 10 mM phosphate buffer (pH 8.0). 8 µg of the double-stranded DNA prepared in (1), and 30 mg of the SG grains thus-equilibrated were reacted in 400 µl of the phosphate buffer for 24 hours at 37°C, whereby the DNA was fixed onto the grains. The reaction system was centrifuged three times with an aqueous solution of 2.5 M sodium chloride to thereby remove the non-carrier bonded DNA. Next, this was processed with 1 ml of a 10 mM Tris-HCl buffer (pH 7.9) at room temperature for 24 hours, whereby the epoxy groups remaining on the surfaces of the SG grains were cleaved.

### (4) Production of Carrier-bonded DNA Probe:

The double-stranded DNA as fixed on 2 mg of the SG grains prepared in (3) was added to 400 ml of a hybridization solution comprising 25 % formamide, 0.75 M sodium chloride, 0.075 M sodium citrate, 1 % bovine serum albumin, 1 % polyvinyl pyrrolidone and 1 % Ficol, processed therein at 80°C for 5 minutes, and washed five times at the same temperature. Thus was formed a carrier-bonded DNA probe, in which the base sequence of Sequence No. 2 was bonded to the SG carrier at the 5'-terminal.

The absorbance at 260 nm of the carrier-bonded probe was measured through spectrophotometry, which verified that the number of the DNA fragments of Sequence No. 2 as bonded to one grain of the carrier is several tens on average.

### Example 2: Reagent in Labeled DNA Probe Method:

### (1) Production of Biotin-labeled DNA Probe:

Using a DNA synthesizer, produced was the following DNA:
5'-CACAAGTTTA TACTCAGGGG-3' (20 mer; Sequence No. 3), which has four G bases next to the 16-mer base sequence complementary to the K-ras sequence except the region to be detected or quantitatively determined.

Next, using a 3'-terminal labeling kit (manufactured by Enzo Co.), 15 µg of the DNA was reacted with 15 µg of biotinylated dUTP (manufactured by Enzo Co.) in the presence of a terminal transferase in a standard reaction buffer, whereby biotin was bonded to the 3'-terminal of the DNA. The non-reacted, biotinylated dUTP was removed in a spin column method using Sephadex G-50, and the intended, biotin-bonded DNA probe was obtained.

### (2) Preparation of Detection Reagents:

Produced was a K-ras mutation detecting kit which comprises the following reagents:
First Reagent:
   10 mM phosphate buffer (pH 8.0) containing 20 mg/ml of the SG grains-bonded DNA probe (this has the DNA of Sequence No. 2) produced in Example 1, and 6 µg/ml of the biotin-labeled DNA probe (this has the DNA of Sequence No. 3) produced in the previous (1).
Second Reagent:
   10 mM phosphate buffer (pH 8.0) containing 100 U/ml of avidin-bonded alkaline phosphatase.
Third Reagent:
   AMPAK (trade name) reagent, manufactured by Dako Co.

### Example 3: Detection of K-ras Mutant Nucleic acid sequence

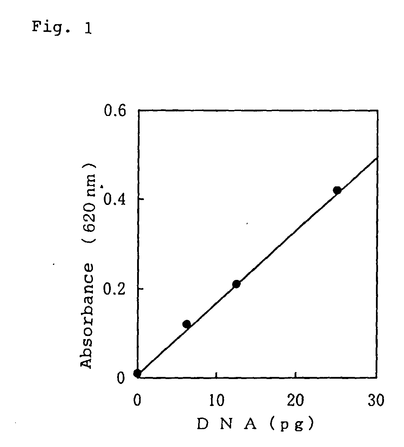

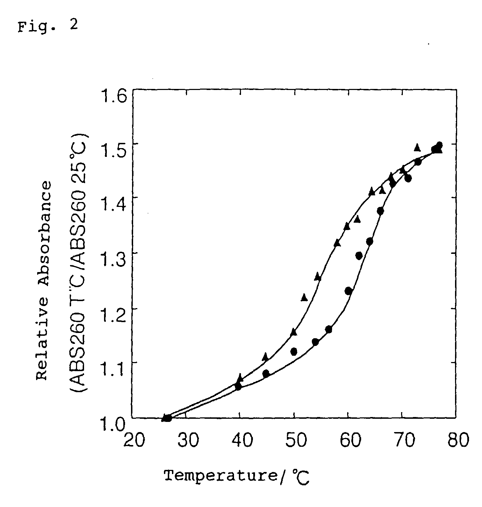

The DNA of Sequence No. 1 was produced using a DNA synthesizer, and its 5'-terminal was labeled with biotin in a cyanoethylphosphoamidite method. 2 mg of the carrier-bonded DNA probe that had been produced in Example 1 was added to 1 ml of a solution comprising 1/50 of a surfactant (neutral detergent containing a nonionic surfactant, manufactured by Kyowa Medex Co., Ltd.), 1 M sodium chloride and 10 mM phosphate buffer (pH 8.0), to which were added a varying amount of the biotin-labeled DNA and 50 µl of 100 U/ml avidin-bonded peroxidase, and kept at room temperature for 60 minutes. Next, the reaction mixture was washed three times through centrifugation with the same solution as above. The carrier was collected, and dispersed in 100 µl of a solvent, to which were added 100 µM MCDP (manufactured by Kyowa Medex Co., Ltd.) and 1 µM hydrogen peroxide, and reacted at 37°C for 30 minutes. The variation in the absorbance at 620 nm before and after the reaction was measured. The data obtained are plotted in Fig. 1.

As shown in Fig. 1, the amount of the DNA can be quantitatively determined.

### Example 4: Detection of One Base Mismatch

Using a DNA synthesizer, produced were a DNA having the following K-ras sequence, which is the same as the bases from the 20^{th} to the 39^{th} of Sequence No. 7:
5'-GTTGGAGCTG GTGGCGTAGG-3' (20-mer; Sequence No. 4); and a DNA having the following sequence, which is synthesized at random, and is not character-related:
5'-CAAGAGTGCC TTGACGATAC-3' (20-mer; Sequence No. 5). These were labeled with biotin in the same manner as in Example 3, and were used herein along with the biotin-labeled DNA of Sequence No. 1 that had been produced in Example 3. 2 mg of the carrier-bonded DNA probe that had been produced in Example 1 was combined with 0.6 µg of any of the above biotin-labeled DNAs to prepare different samples, as shown in Table 5 below. These samples were separately put into the hybridization solution as above, kept therein at 80°C for 10 minutes, and thereafter gradually cooled to 25°C over a period of 3 hours. 50 µl of 100 U/ml avidin-bonded peroxidase was added to each sample, and kept at room temperature for 60 minutes. The peroxidase activity of the carrier in each sample was measured in the same manner as in Example 3. The data obtained are shown in Table 5.

**Table 5**

| DNA-DNA Combination | Difference in Absorbance |
|---|---|
| Sequence No. 1, and Sequence No. 4 | 0.31 |
| Sequence No. 1, and Sequence No. 5 | 0.35 |
| Sequence No. 4, and Sequence No. 5 | 0.04 |

As shown in Table 5, the variation in absorbance was recognized only in the samples of the combination of completely complementary DNAs, but little in the sample of the combination of one base-mismatched DNAs.

### Example 5: Labeled DNA Probe Method

Using a DNA synthesizer, produced were a DNA having the following K-ras mutant sequence:
5'-TGAGTATAAA CTTGTGGTAG TTGGAGCTCG TGGCGTAGGC AAGAGTGCCT TGACGATACA GCTAATTCAG-3' (70-mer; Sequence No. 6 with a point mutation C in position 29);
a DNA having the following K-ras sequence:
5'-TGAGTATAAA CTTGTGGTAG TTGGAGCTGG TGGCGTAGGC AAGAGTGCCT TGACGATACA GCTAATTCAG-3' (70-mer; Sequence No. 7); and
a DNA having the following variant sequence:
5' -TGAGTATAAA CTTGTGGTAC AAGAGTGCCT TGACGATACC AAGAGTGCCT TGACGATACA GCTAATTCAG-3' (70-mer; Sequence No. 8 in which 20 bases from the 20th to the 39th were modified).

Hybridization solutions (having the same composition as in Example 1) were prepared, each being 120 µl in volume and containing any two of those synthetic DNAs of Sequence Nos. 6 to 8 of being 2 µg each. The samples thus-prepared were tested in the manner mentioned below to detect the intended DNA nucleic acid sequence.

120 µl of the first reagent prepared in Example 2, and 120 µl of the sample prepared above were put into a 2 ml micro-tube, then kept at 80°C for 10 minutes, and gradually cooled to 25°C over a period of 3 hours. This was washed two times through centrifugation with 400 µl of 2.5 M sodium chloride solution. Then, 20 µl of the second reagent prepared in Example 2 was added to the residue remaining in the centrifugal tube, and kept at room temperature for 1 hour. This was further washed three times with 400 µl of a solution comprising 1/50 of a surfactant (neutral detergent containing a nonionic surfactant, manufactured by Kyowa Medex Co., Ltd.), 1 M sodium chloride and 10 mM phosphate buffer (pH 7.0), and 500 µl of the third reagent prepared in Example 2 was added to the carrier-containing fraction, and reacted at 37°C for 30 minutes. The absorbance at 492 nm was measured. The data obtained are shown in Table 6 below.

**Table 6**

| DNA-DNA Combination | Difference in Absorbance |
|---|---|
| Sequence No. 6, and Sequence No. 7 | 0.63 |
| Sequence No. 6, and Sequence No. 8 | 0.62 |
| Sequence No. 7, and Sequence No. 8 | 0.02 |

As shown in Table 6, the significant increase in the absorbance was recognized only in the samples of the combination of DNAs both having the entirely same sequence.

### Example 6:

DNAs of Sequence No. 1 and Sequence No. 4 were produced, using a DNA synthesizer, and labeled with biotin in the same manner as in Example 3. 2 mg of the carrier-bonded DNA probe that had been prepared in Example 1, and a varying amount of any of those biotin-labeled DNAs prepared herein were mixed in different molar ratios shown in Table 7 below to prepare different samples. The samples were separately kept in the hybridization solution as above, at 80°C for 10 minutes, and then gradually cooled to 25°C over a period of 3 hours. 50 µl of 100 U/ml avidin-bonded peroxidase was added to each of those samples, and kept at room temperature for 60 minutes. Then, the activity of the peroxidase bonded to the carrier in each sample was measured through absorptiometry in the same manner as in Example 3. The data obtained are shown in Table 7.

**Table 7**

| Molar Concentration of DNA Relative to Probe DNA | | Absorbance |
|---|---|---|
| Sequence 1 | Sequence 2 | |
| 1 | 0 | 0.197 |
| 1 | 10 | 0.147 |
| 1 | 100 | 0.151 |
| 10 | 0 | 0.219 |
| 100 | 0 | 0.314 |

The data in Table 7 verify that the completely complementary sequence can be detected even in the samples in which the amount of the one base mismatched sequence is 100 times by mol the completely complementary sequence.

### Example 7: Production of Labeled, carrier-bonded DNA

In the same manner as in Example 1, produced herein was a carrier-bonded DNA probe in which the base sequence of Sequence No. 2 was bonded to the SG carrier at its 5'-terminal. Next, in the same manner as in Example 2, biotin was bonded to the 3'-terminal of the base sequence in the probe to give a labeled, carrier-bonded DNA.

In this labeled DNA probe, about 0.06 µg of the probe DNA was bonded to 1 mg of the SG grains.

### Example 8: Reagents for Labeled, carrier-bonded DNA Method

A kit comprising the following 4th to 7th reagents was produced.
Fourth Reagent:
   Reagent comprised of 27.8 mg/ml of the labeled, carrier-bonded DNA that had been prepared in Example 7, 2.8 M sodium chloride, 10 mM zinc sulfate, and 300 mM acetate buffer (pH 4.6).
Fifth Reagent:
   Reagent comprised of 300 U/ml S1 nuclease (manufactured by Lifetec Oriental Co.), 150 mM sodium chloride, 0.05 mM zinc sulfate, 50 % glycerol, and 10 mM acetate buffer (pH 4.6).
Sixth Reagent:
   10 mM phosphate buffer (pH 8.0) containing 100 U/ml avidin-bonded alkaline phosphatase.
Seventh Reagent:
   AMPAK (trade name) reagent, manufactured by Dako Co.

### Example 9: Labeled, carrier-bonded DNA Method

2 µg of the DNA of Sequence No. 1 having the K-ras mutant sequence and 2 µg of the DNA of Sequence No. 4 having the K-ras sequence were separately dissolved in 120 µl of water to prepare DNA samples.

120 µl of the 4th reagent prepared in Example 8, and 120 µl of the DNA sample prepared herein were put into a 2 ml micro-tube, then kept at 80°C for 10 minutes, and gradually cooled to 25°C over a period of 3 hours. This was washed two times through centrifugation with 400 µl of 2.5 M sodium chloride solution. Next, 100 µl of the 5th reagent prepared in Example 8 was added to the resulting precipitate fraction, and kept at 37°C or 45°C for 1 hour, whereby the precipitate fraction was processed with S1 nuclease.

After the reaction, this was washed two times through centrifugation with 400 µl of 2.5 M sodium chloride solution, and 20 µl of the 6th reagent prepared in Example 8 was added to the resulting precipitate fraction, and kept at room temperature for 1 hour. This was further washed three times with 400 µl of a solution comprising 1/50 of a surfactant (neutral detergent containing a nonionic surfactant, manufactured by Kyowa Medex Co., Ltd.), 1 M sodium chloride and 10 mM phosphate buffer (pH 7.0), and 500 µl of the 7th reagent prepared in Example 8 was added to the carrier-containing fraction, and reacted at 37°C for 30 minutes. The absorbance at 492 nm was measured.

The data obtained are as follows: Relative to the absorbance, 100%, of the sample comprising the DNA of Sequence No. 1, the absorbance of the sample comprising the DNA of Sequence No. 4 and processed with S1 nuclease at 37°C was 8.2%, while that of the sample comprising the DNA of Sequence No. 4 and processed with S1 nuclease at 45°C was 0%. Those data verify that the completely complementary DNA fragment can be detected and quantitatively determined in the method of the invention, with no detection of the one base mismatched sequence.

### Test Example 1:

Using a DNA synthesizer, produced were the following DNA chain having a K-ras mutant sequence:
5'-TGAGTATAAA CTTGTGGTAG TTGGAGCTCG TGGCGTAGGC AAGAGTGCCT TGACGATACA GCTAATTCAG-3' (70-mer; Sequence No. 6 with a point mutation C in position 29);
the following DNA with 9 G bases added to the sequence complementary to Sequence No. 6:
5'-GGGGGGGGGC TGAATTAGCT GTATCGTCAA GGCACTCTTG CCTACGCCAC GAGCTCCAAC TACCACAAGTT TATACTCA-3' (79-mer; Sequence No. 9); and
the following K-ras sequence:
5'-TGAGTATAAA CTTGTGGTAG TTGGAGCTGG TGGCGTAGGC AAGAGTGCCT TGACGATACA GCTAATTCAG-3' (70-mer; Sequence No. 7).

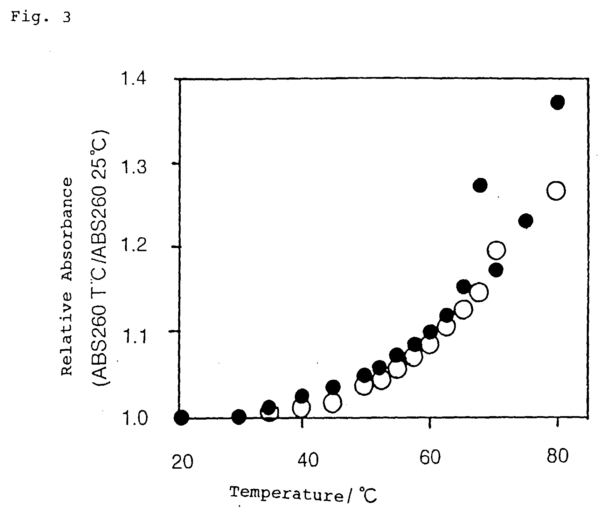

A combination of the probe DNA of Sequence No. 2 and the DNA of Sequence No. 1 (these are completely complementary to each other), and a combination of the probe of Sequence No. 2 and the DNA of Sequence No. 4 (this combination has one base mismatch) were separately added to a hybridization solution comprising 25% formamide, 0.75 M sodium chloride, 0.075 M sodium citrate, 1% bovine serum albumin, 1% polyvinyl pyrrolidone and 1% Ficol, and annealed to obtain DNA dissolution curves, which are shown in Fig. 2. In the same manner, a combination of the probe DNA of Sequence No. 9 and the DNA of Sequence No. 6 (these are completely complementary to each other), and a combination of the probe DNA of Sequence No. 9 and the DNA of Sequence No. 7 (this combination has one base mismatch) were tested to obtain DNA dissolution curves, which are shown in Fig. 3. From these data, it is known that the probe DNA having a length of 70-mer gave little difference in the melting temperature, while the probe having a length of 20-mer gave a difference of about 5°C in the melting temperature. Thus, these data indicate that the length of the probe DNA used has a significant influence on the detection of DNAs. In other words, these data suggest that probe DNAs having a length of less than 70-mer are effective in separating one base mismatched DNAs.

### Test Example 2:

A combination of a DNA chain of Sequence No. 24 (this is a 17-mer, which is composed of a 7-mer sequence completely complementary to the DNA chain of Sequence No. 22 and a 10-mer poly-G sequence at its 5'-terminal) and a DNA chain of Sequence No. 22 with biotin bonded to its 5'-terminal (this is a 7-mer, of which the sequence is completely complementary to the DNA chain of Sequence No. 24); and a combination of the DNA chain of Sequence No. 24 and a DNA chain of Sequence No. 23 with biotin bonded to its 5'-terminal (this is a 7-mer, of which the sequence is incompletely complementary to the DNA chain of Sequence No. 24 in point of one base mismatch between the two) were annealed in the same manner as in Test Example 1 to obtain DNA dissolution curves.

In each system, the absorbance at 260 nm was obtained at 35°C relative to the absorbance at 20°C of being 1. The difference in the relative absorbance between the one base mismatched combination (Sequence No. 23 and Sequence No. 24) and the completely complementary combination (Sequence No. 22 and Sequence No. 24) was obtained.

In the same manner, obtained was the difference in the relative absorbance between a completely complementary combination of Sequence No. 25 (25-mer) with biotin bonded to its 5'-terminal and Sequence No. 27 (25-mer), and a one base mismatched combination of Sequence No. 26 (15-mer) with biotin bonded to its 5'-terminal and Sequence No. 27 (25-mer); between a completely complementary combination of Sequence No. 1 (20-mer) with biotin bonded to its 5'-terminal and Sequence No. 2 (30-mer), and a one base mismatched combination of Sequence No. 4 (20-mer) with biotin bonded to its 5'-terminal and Sequence No. 2 (30-mer); between a completely complementary combination of Sequence No. 28 (25-mer) with biotin bonded to its 5'-terminal and Sequence No. 30 (35-mer), and a one base mismatched combination of Sequence No. 29 (25-mer) with biotin bonded to its 5'-terminal and Sequence No. 30 (35-mer); between a completely complementary combination of Sequence No. 31 (30-mer) with biotin bonded to its 5'-terminal and Sequence No. 33 (40-mer), and a one base mismatched combination of Sequence No. 32 (30-mer) with biotin bonded to its 5'-terminal and sequence No. 33 (40-mer); and between a completely complementary combination of Sequence No. 6 (70-mer) with biotin bonded to its 5'-terminal and Sequence No. 9 (79-mer), and a one base mismatched combination of Sequence No. 7 (70-mer) with biotin bonded to its 5'-terminal and Sequence No. 9 (79-mer).

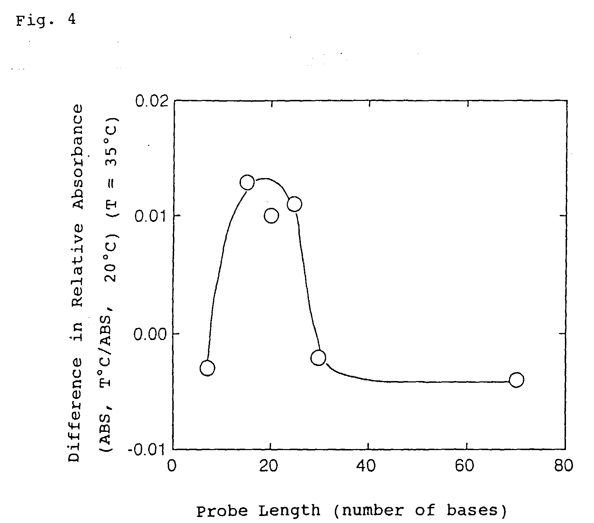

Fig. 4 shows the difference in probe-relative absorbance.

Those data show that the probes having a length of 7 to 70-mer, especially 15 to 25-mer, gave a significant difference in the relative absorbance.

As described in detail hereinabove, the invention has made it possible to simply detect or quantitatively determine intended nucleic acid sequences. Specifically, the technique of the invention is simple and is effective in detecting and quantitatively determining mutant genes at high sensitivity.

While the invention has been described in detail and with reference to specific embodiments thereof, it will be apparent to one skilled in the art that various changes and modifications can be made therein without departing from the spirit and scope thereof.

## Claims

1. A method for detecting or quantitatively determining a single-stranded DNA fragment having a specific nucleic acid sequence in a sample, which comprises stringently hybridizing a carrier-bonded DNA probe that comprises (a) a single-stranded DNA probe having a nucleic acid sequence complementary to the specific nucleic acid sequence of the single-stranded DNA fragment to be detected or quantitatively determined in the sample, and (b) a carrier comprising a substance with a very low adsorbance for DNA, as bonded together via or without a spacer therebetween, with DNA fragments in the sample, followed by detecting or quantitatively determining the DNA fragment as hybridized with the carrier-bonded DNA probe.

2. A method for detecting or quantitatively determining a single-stranded DNA fragment having a specific nucleic acid sequence in a sample, which comprises stringently hybridizing both (A) a carrier-bonded DNA probe that comprises (a) a single-stranded DNA probe having a nucleic acid sequence complementary to the specific nucleic acid sequence of a single-stranded DNA fragment to be detected or quantitatively determined in the sample, and (b) a carrier comprising a substance with a very low adsorbance for DNA, as bonded together via or without a spacer therebetween, and (B) a single-stranded DNA probe having a nucleic acid sequence complementary to a partial nucleic acid sequence except the specific nucleic acid sequence of the single-stranded DNA fragment to be detected or quantitatively determined in the sample, with DNA fragments in the sample, followed by detecting or quantitatively determining the DNA fragments as hybridized with the both probes (A) and (B).

3. A method for detecting or quantitatively determining a single-stranded DNA fragment having a specific nucleic acid sequence in a sample, which comprises hybridizing a carrier-bonded DNA probe that comprises (a) a single-stranded DNA probe having a nucleic acid sequence complementary to the specific nucleic acid sequence of a single-stranded DNA fragment to be detected or quantitatively determined in the sample, and (b) a carrier comprising a substance with a very low adsorbance for DNA, as bonded together via or without a spacer therebetween, with DNA fragments in the sample, then treating it with an enzyme capable of cleaving the single-stranded DNA fragment, and thereafter detecting or quantitatively determining the DNA fragment as hybridized with the carrier-bonded DNA probe.

4. The method for detecting or quantitatively determining a single-stranded DNA fragment having a specific nucleic acid sequence in a sample as claimed in claim 1, in which the DNA fragment is labeled with a labeling compound, and the labeling compound bonding to the DNA fragment as hybridized with the carrier-bonded DNA probe is detected or quantitatively determined to thereby detect or quantitatively determine the hybridized DNA fragment.

5. The method for detecting or quantitatively determining a single-stranded DNA fragment having a specific nucleic acid sequence in a sample as claimed in claim 2, in which the single-stranded DNA probe having a nucleic acid sequence complementary to a partial nucleic acid sequence except the specific nucleic acid sequence of the single-stranded DNA fragment to be detected or quantitatively determined in the sample is a labeled DNA probe, and the labeling compound bonding to said DNA probe is detected or quantitatively determined to thereby detect or quantitatively determine the DNA fragment as hybridized with said labeled probe.

6. The method for detecting or quantitatively determining a single-stranded DNA fragment having a specific nucleic acid sequence in a sample as claimed in claim 3, in which the single-stranded DNA probe moiety in the carrier-bonded DNA probe that comprises (a) a single-stranded DNA probe having a nucleic acid sequence complementary to the specific nucleic acid sequence of the single-stranded DNA fragment to be detected or quantitatively determined in the sample, and (b) a carrier comprising a substance with a very low adsorbance for DNA, as bonded together via or without a spacer therebetween, is labeled with a labeling compound, and the labeling compound bonding to the DNA fragment as hybridized with the labeled, carrier-bonded DNA probe is detected or quantitatively determined to thereby detect or quantitatively determine the hybridized DNA fragment.

7. The method for detecting or quantitatively determining a specific nucleic acid sequence as claimed in any one of claims 1 to 6, in which the nucleic acid sequence to be detected or quantitatively determined is a point mutation gene.

8. The method for detecting or quantitatively determining a specific nucleic acid sequence as claimed in any one of claims 1 to 6, in which the single-stranded DNA probe having a nucleic acid sequence complementary to the nucleic acid sequence to be detected or quantitatively determined in the sample is any of 10- to 30-mers.

9. The method for detecting or quantitatively determining a specific nucleic acid sequence as claimed in any one of claims 1 to 6, in which the spacer is a polyethylene glycol diglycidyl ether, a single-stranded DNA or a single-stranded RNA.

10. The method for detecting or quantitatively determining a specific nucleic acid sequence as claimed in any one of claims 1 to 6, in which the carrier comprising a substance with a very low adsorbance for DNA is in the form of core/shell structured grains of styrene-glycidyl methacrylate.

11. The method for detecting or quantitatively determining a specific nucleic acid sequence as claimed in any one of claims 4 to 6, in which the labeling compound is biotin, and, to detect or quantitatively determine the labeling compound biotin, an avidin-bonded enzyme is bonded to the labeling compound biotin and thereafter the enzymatic activity of the bonded enzyme is detected or quantitatively determined.

12. The method for detecting or quantitatively determining a specific nucleic acid sequence as claimed in claim 10, in which the detection or quantitative determination of the enzymatic activity comprises enzyme-cycling amplification.

13. A carrier-bonded DNA probe, which comprises a 10-meric to 30-meric single-stranded DNA probe having a nucleic acid sequence complementary to the specific nucleic acid sequence of a single-stranded DNA fragment to be detected or quantitatively determined in a sample, and a carrier comprising a substance with a very low adsorbance for DNA, as bonded together via or without a spacer therebetween.

14. The carrier-bonded DNA probe as claimed in claim 13, in which the carrier comprising a substance with a very low adsorbance for DNA is in the form of core/shell structured grains of styrene-glycidyl methacrylate.

15. A reagent kit for detecting or quantitatively determining a single-stranded DNA fragment having a specific nucleic acid sequence in a sample, which comprises (I) a reagent comprising (A) a carrier-bonded DNA probe that comprises (a) a single-stranded DNA probe having a nucleic acid sequence complementary to the specific nucleic acid sequence of a single-stranded DNA fragment to be detected or quantitatively determined in the sample, and (b) a carrier comprising a substance with a very low adsorbance for DNA as bonded together via or without a spacer therebetween, and (B) a labeled DNA probe that comprises (c) a single-stranded DNA probe having a nucleic acid sequence complementary to a partial nucleic acid sequence except the specific nucleic acid sequence of the single-stranded DNA fragment to be detected or quantitatively determined in the sample, and (d) a labeling compound as bonded together, and (II) a reagent for detecting or quantitatively determining the labeling compound.

16. The reagent kit for detecting or quantitatively determining a specific nucleic acid sequence as claimed in claim 15, in which the carrier-bonded DNA probe has a length of 10- to 30-mers, and the labeled DNA probe has a length of 10- to 30-mers.

17. The reagent kit for detecting or quantitatively determining a specific nucleic acid sequence as claimed in claim 15 or 16, in which the labeling compound for the labeled DNA probe is biotin, and the reagent for detecting or quantitatively determining the labeling compound biotin is a reagent for detecting or quantitatively determining the enzymatic activity of an avidin-bonded enzyme.

18. The reagent kit for detecting or quantitatively determining a specific nucleic acid sequence as claimed in claim 17, in which the avidin-bonded enzyme is an alkaline phosphatase, and the reagent for detecting or quantitatively determining the enzymatic activity of the avidin-bonded enzyme comprises NADP, INT-violet, NADH, ethanol, diaphorase, and alcohol dehydrogenase.

19. A reagent kit for detecting or quantitatively determining a single-stranded DNA fragment having a specific nucleic acid sequence in a sample, which comprises (I) a reagent comprising a labeled, carrier-bonded DNA probe that comprises (a) a labeled single-stranded DNA probe comprising a nucleic acid sequence complementary to the specific nucleic acid sequence of a single-stranded DNA fragment to be detected or quantitatively determined in the sample and labeling compound as bonded together, and (b) a carrier comprising a substance with a very low adsorbance for DNA, as bonded together via or without a spacer therebetween, (II) a reagent comprising an enzyme capable of cleaving the single-stranded DNA, and (III) a reagent for detecting or quantitatively determining the labeling compound.

20. The reagent kit for detecting or quantitatively determining a specific nucleic acid sequence as claimed in claim 19, in which the labeled, carrier-bonded DNA probe is any of 10- to 30-mers.

21. The reagent kit for detecting or quantitatively determining a specific nucleic acid sequence as claimed in claim 19 or 20, in which the labeling compound for the labeled, carrier-bonded DNA probe is biotin, and the reagent for detecting or quantitatively determining the labeling compound biotin is a reagent for detecting or quantitatively determining the enzymatic activity of an avidin-bonded enzyme.

22. The reagent kit for detecting or quantitatively determining a specific nucleic acid sequence as claimed in claim 21, in which the avidin-bonded enzyme is an alkaline phosphatase, and the reagent for detecting or quantitatively determining the enzymatic activity of the avidin-bonded enzyme comprises NADP, INT-violet, NADH, ethanol, diaphorase, and alcohol dehydrogenase.

23. A labeled, carrier-bonded DNA probe, which comprises (a) a labeled single-stranded DNA probe comprising a nucleic acid sequence complementary to the specific nucleic acid sequence of a single-stranded DNA fragment to be detected or quantitatively determined in a sample, and a labeling compound as bonded together, and (b) a carrier comprising a substance with a very low adsorbance for DNA, as bonded together via or without a spacer therebetween.

24. The method for detecting or quantitatively determining a single-stranded DNA fragment having a specific nucleic acid sequence in a sample as claimed in claim 1, 2, or 3, wherein the single-stranded DNA fragment is a DNA fragment containing the specific nucleic acid sequence obtained by extracting DNAs from cells and cleaving the DNAs with a specific restriction endonuclease.


SEQUENCE LISTING

(1) GENERAL INFORMATION:

(i) APPLICANT:
(A) NAME: Kyowa Medex Co., Ltd.
(B) STREET: 1-1, Irifune 2-chome
(C) CITY: Chuo-ku, Tokyo
(E) COUNTRY: Japan
(F) POSTAL CODE (ZIP): 104

(ii) TITLE OF INVENTION: METHOD FOR DETERMINATION OF SPECIFIC
NUCLEIC ACID SEQUENCE, AND A REAGENT THEREFOR

(iii) NUMBER OF SEQUENCES: 33

(iv) COMPUTER READABLE FORM:
(A) MEDIUM TYPE: Diskette-3.5 inch, 1440 Kb storage
(B) COMPUTER: IBM PC compatible
(C) OPERATING SYSTEM: PC-DOS/MS-DOS
(D) SOFTWARE: PatentIn Release #1.0, Version #1.25

(vi) CURRENT APPLICATION DATA:
(A) APPLICATION NUMBER: EP 97 11 9495.6
(B) FILING DATE: 07-NOV-1997
(v) PRIOR APPLICATION DATA:
(A) APPLICATION NUMBER: JP296963/96
(B) FILING DATE:08-NOV-1996

(2) INFORMATION FOR SEQ ID NO:1:

(i) SEQUENCE CHARACTERISTICS:
(A) LENGTH: 20 base
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:1:

GTTGGAGCTC GTGGCGTAGG 20

(2) INFORMATION FOR SEQ ID NO:2:

(i) SEQUENCE CHARACTERISTICS:
(A) LENGTH: 30 base
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:2:

GGGGGGGGGG CCTACGCCAC GAGCTCCAAC 30

(2) INFORMATION FOR SEQ ID NO:3:

(i) SEQUENCE CHARACTERISTICS:
(A) LENGTH: 20 base
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:3:

CACAAGTTTA TACTCAGGGG 20

(2) INFORMATION FOR SEQ ID NO:4:

(i) SEQUENCE CHARACTERISTICS:
(A) LENGTH: 20 base
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:4:

GTTGGAGCTG GTGGCGTAGG 20

(2) INFORMATION FOR SEQ ID NO:5:

(i) SEQUENCE CHARACTERISTICS:
(A) LENGTH: 20 base
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:5:

CAAGAGTGCC TTGACGATAC 20

(2) INFORMATION FOR SEQ ID NO:6:

(i) SEQUENCE CHARACTERISTICS:
(A) LENGTH: 70 base
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:6:

TGAGTATAAA CTTGTGGTAG TTGGAGCTCG TGGCGTAGGC AAGAGTGCCT TGACGATACA 60

GCTAATTCAG 70

(2) INFORMATION FOR SEQ ID NO:7:

(i) SEQUENCE CHARACTERISTICS:
(A) LENGTH: 70 base
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:7:

TGAGTATAAA CTTGTGGTAG TTGGAGCTGG TGGCGTAGGC AAGAGTGCCT TGACGATACA 60

GCTAATTCAG 70

(2) INFORMATION FOR SEQ ID NO:8:

(i) SEQUENCE CHARACTERISTICS:
(A) LENGTH: 70 base
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:8:

TGAGTATAAA CTTGTGGTAC AAGAGTGCCT TGACGATACC AAGAGTGCCT TGACGATACA 60

GCTAATTCAG 70

(2) INFORMATION FOR SEQ ID NO:9:

(i) SEQUENCE CHARACTERISTICS:
(A) LENGTH: 79 base
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:9:

GGGGGGGGGC TGAATTAGCT GTATCGTCAA GGCACTCTTG CCTACGCCAC GAGCTCCAAC 60

TACCACAAGT TTATACTCA 79

(2) INFORMATION FOR SEQ ID NO:10:

(i) SEQUENCE CHARACTERISTICS:
(A) LENGTH: 20 base
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:10:

GGATAGGCTG ACGTCTACCT 20

(2) INFORMATION FOR SEQ ID NO:11:

(i) SEQUENCE CHARACTERISTICS:
(A) LENGTH: 20 base
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:11:

GAGCCATCCT GCCCACCCCA 20

(2) INFORMATION FOR SEQ ID NO:12:

(i) SEQUENCE CHARACTERISTICS:
(A) LENGTH: 20 base
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:12:

CCAAGAGGGA CGGGAACCTC 20

(2) INFORMATION FOR SEQ ID NO:13:

(i) SEQUENCE CHARACTERISTICS:
(A) LENGTH: 20 base
(B) TYPE: nucleic acid
(C) STRANDEDNESS: single

(D) TOPOLOGY: linear

(ii) MOLECULE TYPE: Other nucleic acid, synthetic DNA

(xi) SEQUENCE DESCRIPTION: SEQ ID NO:13:

ACCCTCGTTT CCGTACAGAG 20

(2) INFORMATION FOR SEQ ID NO:14:

(i) SEQUENCE CHARACTERISTICS:
(A) LENGTH: 20 base
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:14:

GCTGAGCCCA GGACCGGTCT 20

(2) INFORMATION FOR SEQ ID NO:15:

(i) SEQUENCE CHARACTERISTICS:
(A) LENGTH: 20 base
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:15:

TTCTTGTGGC AGCTTAAGTT 20

(2) INFORMATION FOR SEQ ID NO:16:

(i) SEQUENCE CHARACTERISTICS:
(A) LENGTH: 21 base
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:16:

TTCCTGTGGC AGCCTAAGAG G 21

(2) INFORMATION FOR SEQ ID NO:17:

(i) SEQUENCE CHARACTERISTICS:
(A) LENGTH: 21 base
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:17:

GTTTCTTGGA GCAGGTTAAA C 21

(2) INFORMATION FOR SEQ ID NO:18:

(i) SEQUENCE CHARACTERISTICS:
(A) LENGTH: 21 base
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:18:

AGTTCCTGGA AAGACTCTTC T 21

(2) INFORMATION FOR SEQ ID NO:19:

(i) SEQUENCE CHARACTERISTICS:
(A) LENGTH: 21 base
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:19:

GGTTGCTGGA AAGACGCGTC C 21

(2) INFORMATION FOR SEQ ID NO:20:

(i) SEQUENCE CHARACTERISTICS:
(A) LENGTH: 21 base
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:20:

GATTCCCCGC AGAGGATTTC G 21

(2) INFORMATION FOR SEQ ID NO:21:

(i) SEQUENCE CHARACTERISTICS:
(A) LENGTH: 28 base
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:21:

CACCTGCAGT GCGGAGCTCC AACTGGTA 28

(2) INFORMATION FOR SEQ ID NO:22:

(i) SEQUENCE CHARACTERISTICS:
(A) LENGTH: 7 base
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:22:

GCTCGTG 7

(2) INFORMATION FOR SEQ ID NO:23:

(i) SEQUENCE CHARACTERISTICS:
(A) LENGTH: 7 base
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:23:

GCTGGTG 7

(2) INFORMATION FOR SEQ ID NO:24:

(i) SEQUENCE CHARACTERISTICS:
(A) LENGTH: 17 base
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:24:

GGGGGGGGGG CACGAGC 17

(2) INFORMATION FOR SEQ ID NO:25:

(i) SEQUENCE CHARACTERISTICS:
(A) LENGTH: 15 base
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:25:

TGGAGCTCGT GGCGT 15

(2) INFORMATION FOR SEQ ID NO:26:

(i) SEQUENCE CHARACTERISTICS:
(A) LENGTH: 15 base
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:26:

TGGAGCTGGT GGCGT 15

(2) INFORMATION FOR SEQ ID NO:27:

(i) SEQUENCE CHARACTERISTICS:
(A) LENGTH: 25 base
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:27:

GGGGGGGGGG ACGCCACGAG CTCCA 25

(2) INFORMATION FOR SEQ ID NO:28:

(i) SEQUENCE CHARACTERISTICS:
(A) LENGTH: 25 base
(B) TYPE: nucleic acid
(C) STRANDEDNESS: single

(D) TOPOLOGY: linear

(ii) MOLECULE TYPE: Other nucleic acid, synthetic DNA

(xi) SEQUENCE DESCRIPTION: SEQ ID NO:28:

GTAGTTGGAG CTCCTGGCGT AGGCA 25

(2) INFORMATION FOR SEQ ID NO:29:

(i) SEQUENCE CHARACTERISTICS:
(A) LENGTH: 25 base
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:29:

GTAGTTGGAG CTGGTGGCGT AGGCA 25

(2) INFORMATION FOR SEQ ID NO:30:

(i) SEQUENCE CHARACTERISTICS:
(A) LENGTH: 35 base
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:30:

GGGGGGGGGG TGCCTACGCC ACGAGCTCCA ACTAC 35

(2) INFORMATION FOR SEQ ID NO:31:

(i) SEQUENCE CHARACTERISTICS:
(A) LENGTH: 30 base
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:31:

TGGTAGTTGG AGCTCGTGGC GTAGGCAAGA 30

(2) INFORMATION FOR SEQ ID NO:32:

(i) SEQUENCE CHARACTERISTICS:
(A) LENGTH: 30 base
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:32:

TGGTAGTTGG AGCTGGTGGC GTAGGCAAGA 30

(2) INFORMATION FOR SEQ ID NO:33:

(i) SEQUENCE CHARACTERISTICS:
(A) LENGTH: 40 base
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:33:

GGGGGGGGGG TCTTGCCTAC GCCACGAGCT CCAACTACCA 40